# EUROPÄISCHE PATENTSCHRIFT

(11) **EP 3 270 809 B1**
(45) Veröffentlichungstag und Bekanntmachung des Hinweises auf die Patenterteilung: **02.06.2021**
(21) Anmeldenummer: 16712297.7
(22) Anmeldetag: 18.03.2016
(51) Int. Cl.: A61B 18/14, A61B 17/11, A61B 18/00, A61B 17/00, A61B 17/115, A61B 17/072

(54) **CHIRURGISCHES GEWEBEFUSIONSINSTRUMENT UND STÜTZSTRUKTUR HIERFÜR**
SURGICAL TISSUE FUSION INSTRUMENT AND SUPPORT STRUCTURE FOR SAME
INSTRUMENT CHIRURGICAL DE FUSION TISSULAIRE ET STRUCTURE DE SUPPORT CORRESPONDANTE

(30) Priorität: 20.03.2015 DE 102015205056
(43) Veröffentlichungstag der Anmeldung: 24.01.2018
(73) Patentinhaber: Aesculap AG, 78532 Tuttlingen (DE)
(72) Erfinder: BARGON, Rainer, 78532 Tuttlingen (DE); BLENDER, Bernd, 78570 Mühlheim a.d. Donau (DE); EICK, Stefan, 78532 Tuttlingen (DE); HAFNER, Nikolaus, 78532 Tuttlingen (DE); HEIZMANN, Patrick, 78183 Hüfingen (DE); HERNER, Eugen, 78054 Villingen-Schwenningen (DE); HUBER, Christian, 78570 Mühlheim (DE); MERCKLE, Christof, 68199 Mannheim (DE); ODERMATT, Erich, 8200 Schaffhausen (CH); ROTHWEILER, Christoph, 78166 Donaueschingen (DE)
(74) Vertreter: Patentanwälte Ruff, Wilhelm, Beier, Dauster & Partner mbB
(86) Internationale Anmeldenummer: PCT/EP2016/055990
(87) Internationale Veröffentlichungsnummer: WO 2016/150861

(56) Entgegenhaltungen:
- WO-A2-2008/082444
- DE-A1-102010 020 664
- US-A1- 2004 143 263
- US-A1- 2004 210 282
- US-A1- 2005 021 026
- US-A1- 2005 059 997
- US-A1- 2012 022 531

## Beschreibung

Die Erfindung betrifft ein zirkuläres chirurgisches Gewebefusionsinstrument gemäß Anspruch 1. Weitere bevorzugte Ausführungsformen können den abhängigen Ansprüchen entnommen werden.

Ein derartiges chirurgisches Gewebefusionsinstrument ist aus der DE 10 2010 020 664 A1 bekannt. Das Gewebefusionsinstrument ist als zirkuläres Instrument ausgeführt mit einem Ambossteil, der linearbeweglich in einem Basisteil verfahrbar ist. Der Ambossteil und ein Instrumentenkopf des Basisteils weisen einander zugewandte Kontaktflächen auf, denen Elektrodenanordnungen zugeordnet sind, um einen Wärmeeintrag auf zwischen den Kontaktflächen geklemmte biologische Gewebeabschnitte ausüben zu können, der eine Gewebefusion der biologischen Gewebeabschnitte bewirkt. Zwischen den gegenüberliegenden Kontaktflächen des Ambossteils und des Instrumentenkopfs des Basisteils ist als Stützstruktur eine Scheibe angeordnet, die einen Ambossschaft des Ambossteils ringförmig umgibt. Die Scheibe stellt eine Stützstruktur dar, die bei der Gewebefusion zwischen den biologischen Gewebeabschnitten positioniert ist und aus einem Material gestaltet ist, das die Gewebeverbindung unterstützt oder fördert und das medizinisch verträglich ist.

Dokumente US2012022531 A1, US2004210282 A1, US2005021026 A1, US2004143263 A1, WO2008082444 A2 und US2005059997 A1 offenbaren weiteren einschlägigen Stand der Technik.

Aufgabe der Erfindung ist es, ein chirurgisches Gewebefusionsinstrument und eine Stützstruktur der eingangs genannten Art zu schaffen, die eine weiter verbesserte Verbindung der biologischen Gewebeabschnitte miteinander ermöglichen.

Diese Aufgabe wird durch die Merkmale des Anspruchs 1 gelöst. Das erfindungsgemäße chirurgische Gewebefusionsinstrument schafft eine stoffschlüssige Verbindung zwischen den biologischen Gewebeabschnitten vorzugsweise ausschließlich durch Strukturänderungen des biologischen Materials der Gewebeabschnitte, d.h. insbesondere ohne zusätzliche mechanische Verbindungsmittel wie Klammern oder Fäden. Das erfindungsgemäße Gewebefusionsinstrument bewirkt demzufolge vorzugsweise eine klammerfreie Gewebefusion. Die Stützstruktur wird erfindungsgemäß bei zirkulären Gewebefusionsinstrumenten eingesetzt. Es kann auch vorgesehen sein, ein Gewebefusionsinstrument zusätzlich mit einer Klammerapplikationsfunktion zu versehen, um die Gewebeverbindung durch eine mechanisch über Klammern erzeugte Klammernaht zu unterstützen. Erfindungsgemäß ist die Stützstruktur bei einem zirkulären Gewebefusionsinstrument zur Verbindung von biologischen Gewebeabschnitten einsetzbar, die als Hohlorgane gestaltet sind.

Die Stützstruktur weist ein medizinisch verträgliches Material auf oder ist aus einem medizinisch verträglichen Material hergestellt und unterstützt oder fördert die Gewebefusion zwischen den Gewebeabschnitten. Die Stützstruktur kann bei bevorzugten Ausführungsformen resorbierbar ausgeführt sein. Zusätzlich weist die Stützstruktur wenigstens eine weitere körperliche Funktionsstruktur auf, die die Verbindung der Gewebeabschnitte verbessert. Die körperliche Funktionsstruktur kann rein mechanisch ausgeführt sein. Alternativ oder ergänzend kann sie physikalische oder chemische Eigenschaften haben, die abhängig von Umgebungs- oder Funktionsparametern aktivierbar sind.

Das im vorherigen Absatz erwähnte medizinisch verträgliche Material kann ausgewählt sein aus der Gruppe umfassend oder bestehend aus synthetisches Polymer, Biopolymer (natürlich vorkommendes Polymer), technisches Biopolymer (industriell hergestelltes Biopolymer), Protein, extrazelluläres Protein, Serumprotein, Glykoprotein, Polyaminosäure, Polyhomoaminosäure, Polyheteroaminosäure, Polysaccharid, Mucopolysaccharid und Mischungen davon.

Bei dem synthetischen Polymer kann es sich grundsätzlich um ein resorbierbares Polymer oder um nicht resorbierbares Polymer handeln. Ein bevorzugtes resorbierbares Polymer kann beispielsweise ausgewählt sein aus der Gruppe umfassend oder bestehend aus Polyglycolid, Polylactid, Polytrimethylencarbonat, Poly-ε-Caprolacton, Poly-3-hydroxybutyrat, Poly-4-hydroxybutyrat, Poly-5-hydroxybutyrat, Poly-6-hydroxybutyrat und Mischungen davon. Ein bevorzugtes nicht resorbierbares Polymer stellt ein fluorhaltiges, insbesondere perfluorhaltiges, Polymer, wie beispielsweise Polytetrafluorethylen, dar.

Das Protein kann ausgewählt sein aus der Gruppe umfassend oder bestehend aus Kollagen, Gelatine, Elastin, Retikulin, Laminin, Fibronektin, Fibrillin, Albumin, Derivate davon, Peptidfragmente davon, Untereinheiten davon und Mischungen davon.

Bei dem Protein kann es sich insbesondere um Kollagen handeln, welches ausgewählt ist aus der Gruppe umfassend oder bestehend aus Kollagen Typ I, Kollagen Typ II, Kollagen Typ III, Kollagen Typ VI, Derivate davon, Peptidfragmente davon, Untereinheiten davon und Mischungen davon.

Das Polysaccharid kann ausgewählt sein aus der Gruppe umfassend oder bestehend aus Stärke, modifizierte Stärke, Amylose, Amylopektin, Dextran, Hyaluronsäure, Heparin, Heparansulfat, Chondroitin-4-sulfat, Chondroitin-6-sulfat, Dermatansulfat, Keratansulfat, Derivate davon und Mischungen davon.

In Ausgestaltung der Erfindung weist die körperliche Funktionsstruktur Durchtritte oder Materialschwächungen in der Stützstruktur auf, die in Bereichen positioniert sind, in denen der Wärmeeintrag der Wärmeerzeugungsmittel die Stützstruktur und die Gewebeabschnitte durchdringt. Die Wärmeerzeugung im Bereich der Verbindungsstelle der biologischen Gewebeabschnitte erzeugt eine gewünschte stoffschlüssige Verbindung zwischen den Gewebeabschnitten nach Art einer Verschweißung. Die Durchtritte oder Materialschwächungen in der Stützstruktur bewirken in diesen Bereichen einen verbesserten Durchtritt von Energiestrahlung einerseits und zudem eine direkte Kontaktierung der Gewebeabschnitte im Bereich der Durchtritte, falls die Stützstruktur zwischen den Gewebeabschnitten positioniert ist. Unter den Wärmeerzeugungsmitteln sind alle Arten von Energieeintragmitteln zu verstehen, die entweder direkt Wärmeenergie zu der Verbindungsstelle zuführen oder die Energiewellen zuführen, die an der Verbindungsstelle selbst zu einer Wärmeerzeugung in dem wenigstens einen Gewebeabschnitt führen. Als Wärmeerzeugungsmittel sind insbesondere Hochfrequenz- oder Radiofrequenz-Elektroden vorgesehen, die in den Greifstrukturen integriert sind. Alternativ können auch Lasereinheiten, Sonotroden, Mikrowellengeneratoren, Plasmageneratoren, Widerstandsheizgeräte wie Kauter oder eine Kombination aus zwei oder mehreren der genannten Wärmeerzeugungseinrichtungen als Wärmeerzeugungsmittel vorgesehen bzw. in den Greifstrukturen integriert sein.

In weiterer Ausgestaltung der Erfindung sind die Durchtritte als zirkulär erstreckte Langlöcher gestaltet.

In weiterer Ausgestaltung der Erfindung sind die Durchtritte als Perforationen ausgeführt. Die Perforationen bilden eine Vielzahl von gleichmäßig über die entsprechenden Bereiche der Stützstruktur verteilten kleinen Löchern.

In weiterer Ausgestaltung der Erfindung ist die körperliche Funtionsstruktur als in Bewegungsrichtung der Greifstrukturen erstreckter Randsteg gestaltet, der den Bereich der Verbindungsstelle der Gewebeabschnitte außen flankiert. Der Randsteg ist bei einer scheiben- oder ringförmigen Stützstruktur ringförmig am Außenrand der Stützstruktur vorgesehen und bewirkt eine Versteifung und Stützung der miteinander verbundenen Gewebeabschnitte, insbesondere entsprechend miteinander verbundener biologischer Hohlorgane.

In einer weiteren Ausgestaltung umfasst die körperliche Funktionsstruktur wenigstens einen Speicherraum zur Aufnahme eines flüssigen oder fließfähigen Zusatzstoffs. Bevorzugt umfasst die körperliche Funktionsstruktur wenigstens einen Speicherraum, welcher einen flüssigen oder fließfähigen Zusatzstoff enthält. Der wenigstens eine Speicherraum ist in der Stützstruktur integriert. Entsprechende, den Greifstrukturen zugewandte Wandungen des Speicherraums reißen vorzugsweise auf, sobald die Greifstrukturen für eine Aktivierung eines Gewebefusionsvorgangs zusammengeführt werden.

In einer weiteren Ausgestaltung weist die körperliche Funktionsstruktur Profilierungen, insbesondere in Form von kantigen oder eckigen Aus- oder Einformungen, auf, die auf wenigstens einer zu einer Greifstruktur gerichteten Stirnfläche der Stützstruktur vorgesehen sind. Die Profilierungen verbessern die Verbindung der Stützstruktur zu wenigstens einem Gewebeabschnitt. Zudem können die Profilierungen dazu vorgesehen sein, bei einer Druckbeaufschlagung durch die Greifstrukturen im Bereich der wenigstens einen Stirnfläche der Stützstruktur deformiert zu werden und ein Aufreißen der Stützstruktur zu bewirken, wodurch insbesondere ein flüssiger oder fließfähiger Zusatzstoff aus wenigstens einem Speicherraum freigegeben wird. Dies führt je nach Ausführung zu einer Zugabe des Zusatzstoffes auf einer Seite oder auf beiden Seiten des wenigstens einen Gewebeabschnittes.

Der Ausdruck "Zusatzstoff" kann einen Zusatzstoff (Singular) oder eine Mehrzahl von Zusatzstoffen (Plural), d.h. zwei oder mehr Zusatzstoffe, bedeuten.

Der Zusatzstoff wird vorzugsweise in flüssiger, insbesondere als Lösung, Suspension oder Emulsion, bevorzugt als wässrige Lösung, wässrige Suspension oder wässrige Emulsion, oder fließfähiger, d.h. insbesondere partikulärer, pastöser, geschmolzener oder gelförmiger, bevorzugt hydrogelförmiger, Form vorgehalten. Dadurch kann der Zusatzstoff bei einem Wärmeeintrag während eines Gewebefusionsvorganges im Bereich der zu verbindenden Gewebeabschnitte insbesondere unter Druck- und/oder Temperatureinfluss zufließen.

Der Zusatzstoff kann ausgewählt sein aus der Gruppe umfassend oder bestehend aus Salz wie anorganisches Salz, Wachs, Fett, Fettsäure, Alkohol, synthetisches Polymer, Biopolymer (natürlich vorkommendes Polymer), technisches Biopolymer (industriell hergestelltes Biopolymer), Protein, extrazelluläres Protein, Serumprotein, Glykoprotein, Polyaminosäure, Polyhomoaminosäure, Polyheteroaminosäure, Oligopeptid, Aminosäure, Polysaccharid, Mucopolysaccharid, Oligosaccharid, Monosaccharid, Lipid, Glykolipid, Medikament, medizinischer bzw. pharmazeutischer Wirkstoff, Wachstumsfaktor, Cyanoacrylat und Mischungen davon.

Das Salz kann ausgewählt sein aus der Gruppe umfassend oder bestehend aus Alkalimetallhalogenid, Erdalkalimetallhalogenid, Phosphat, Alkalimetallphosphat, Erdalkalimetallphosphat und Mischungen davon.

Das Salz kann insbesondere ausgewählt sein aus der Gruppe umfassend oder bestehend aus Natriumchlorid, Kaliumchlorid, Bariumchlorid, Magnesiumchlorid, Calciumchlorid, Natriumphosphat, Kaliumphosphat, Bariumphosphat, Magnesiumphosphat, Calciumphosphat, Mischphosphate davon und Mischungen davon.

Das synthetische Polymer kann beispielsweise ausgewählt sein aus der Gruppe umfassend oder bestehend aus Polyglycolid, Polylactid, Polytrimethylencarbonat, Poly-ε-Caprolacton, Poly-3-hydroxybutyrat, Poly-4-hydroxybutyrat, Poly-5-hydroxybutyrat, Poly-6-hydroxybutyrat und Mischungen davon.

Das Protein kann ausgewählt sein aus der Gruppe umfassend oder bestehend aus Kollagen, Gelatine, Elastin, Retikulin, Laminin, Fibronektin, Fibrillin, Albumin, Derivate davon, Peptidfragmente davon, Untereinheiten davon und Mischungen davon.

Bei dem Protein kann es sich insbesondere um Kollagen handeln, welches ausgewählt ist aus der Gruppe umfassend oder bestehend aus Kollagen Typ I, Kollagen Typ II, Kollagen Typ III, Kollagen Typ VI, Derivate davon, Peptidfragmente davon, Untereinheiten davon und Mischungen davon.

Das Polysaccharid kann ausgewählt sein aus der Gruppe umfassend oder bestehend aus Stärke, modifizierte Stärke, Amylose, Amylopektin, Dextran, Hyaluronsäure, Heparin, Heparansulfat, Chondroitin-4-sulfat, Chondroitin-6-sulfat, Dermatansulfat, Keratansulfat, Derivate davon und Mischungen davon.

Das Medikament kann ausgewählt sein aus der Gruppe umfassend oder bestehend aus Antibiotikum, Zytostatikum, Spasmolytikum, Thrombozytenaggregations-Hemmer, Antikoagulans, Hormon, Magen-Darm-Therapeutikum, Lokalanästhetikum, Antihypertensivum, Antiphlogistikum, Analgetikum und Mischungen davon.

Der medizinische bzw. pharmazeutische Wirkstoff kann ausgewählt sein aus der Gruppe umfassend oder bestehend aus antimikrobieller, insbesondere antibiotischer, Wirkstoff, blutstillender Wirkstoff (Hämostyptikum), entzündungshemmender Wirkstoff, wundheilungsfördernder Wirkstoff, schmerzstillender Wirkstoff, wachstumsanregender Wirkstoff und Mischungen davon.

Der antimikrobielle Wirkstoff kann ausgewählt sein aus der Gruppe umfassend oder bestehend aus Silber, Silbersalz, Antibiotikum, Polyhexamethylenbiguanid und Mischungen davon.

Der Wachstumsfaktor kann ausgewählt sein aus der Gruppe umfassend oder bestehend aus Fibroblasten-Wachstumsfaktor (FGF, Fibroblast Growth Factor), transformierender Wachstumsfaktor (TGF, Transforming Growth Factor), PDGF (Platelet-Derived Growth Factor), epidermaler Wachstumsfaktor (EGF, Epidermal Growth Factor), GMCSF (Granulocyte-Macrophage Colony Stimulating Factor), vaskulärer endothelialer Wachstumsfaktor (VEGF, Vascular Endothelial Growth Factor), insulinähnlicher Wachstumsfaktor (IGF, Insulin-like Growth Factor), Hepatozyten-Wachstumsfaktor (HGF, Hepatocyte Growth Factor), Interleukin, Nervenwachstumsfaktor (NGF, Nerve Growth Factor), hämatopoetischer Wachstumsfaktor und Mischungen davon.

In einer weiteren Ausgestaltung handelt es sich bei dem Zusatzstoff um einen gewebekontaktverbessernden Zusatzstoff. Insbesondere kann es sich bei dem Zusatzstoff um einen Zusatzstoff handeln, welcher dazu ausgebildet ist, den Gewebekontakt zu einem Sensor oder einer Sensoranordnung des chirurgischen Gewebefusionsinstruments zu verbessern. Ein derartiger Zusatzstoff kann insbesondere ein Immersionsmedium, wie beispielsweise Glycerin, Hydrogele oder Öle mit einem definierten Brechungsindex, sein.

In einer weiteren Ausgestaltung handelt es sich bei dem Zusatzstoff um einen Zusatzstoff, welcher dazu ausgebildet ist, eine bildgebende Auswertung der miteinander zu verbindenden biologischen Gewebeabschnitte oder von bereits miteinander verbundenen biologischen Gewebeabschnitten zu verbessern. Ein geeigneter Zusatzstoff kann insbesondere als Kontaktmaterial zur besseren Einkopplung von beispielsweise Ultraschall zur bildgebenden Auswertung ausgebildet sein. Geeignete Zusatzstoffe stellen beispielsweise Hydrogele aus Carbomeren oder deren Derivaten, pflanzliche Lipogele (Oleogele), synthetische Lipogele (Olegele), mineralische Lipogele (Oleogele), Hyaluronsäure oder biologische Gele wie Aloe Vera dar.

In einer weiteren Ausgestaltung handelt es sich bei dem Zusatzstoff um einen Zusatzstoff, welcher dazu ausgebildet ist, einen Energieeintrag und damit einen Wärmeeintrag in die miteinander zu verbindenden biologischen Gewebeabschnitte zu verbessern. Ein geeigneter Zusatzstoff stellt/stellen beispielsweise Glycerin, biologische Öle, synthetische Öle, mineralische Öle, Hydrogele oder Lipogele (Oleogele) dar.

In einer weiteren Ausgestaltung handelt es sich bei dem Zusatzstoff um einen energiewandelnden oder -absorbierenden Zusatzstoff, welcher dazu ausgebildet ist, eine positionierte Wandlung von einer Energieform, wie beispielsweise Lichtenergie, in Wärmeenergie zu bewirken. Ein geeigneter Zusatzstoff können beispielsweise metallische oder metallisierte Partikel wie Silberpartikel, Kohlenstoffpartikel oder weitere, absorbierende Materialien oder Nanostrukturen sein. Der Zusatzstoff kann in Form von Partikeln, Emulsionen, Schmelzen oder in gelöster Form appliziert werden.

In einer weiteren Ausgestaltung handelt es sich bei dem Zusatzstoff um einen energiekoppelnden Zusatzstoff, welcher insbesondere dazu ausgebildet ist, die Einbringung von induktiver Energie zu bewirken. Bei einem solchen Zusatzstoff kann es sich beispielsweise um ferromagnetische Partikel, Graphit oder kohlenstoffhaltige Materialien handeln.

In einer weiteren Ausgestaltung handelt es sich bei dem Zusatzstoff um einen gewebsstrukturmarkierenden Zusatzstoff, welcher dazu ausgebildet ist, eine Markierung der miteinander zu verbindenden biologischen Gewebeabschnitte oder von bereits miteinander verbundenen biologischen Gewebeabschnitten zu bewirken. Ein geeigneter Zusatzstoff stellt beispielsweise ein Fluoreszenzfarbstoff dar. Die Verwendung von Fluoreszenzfarbstoffen hat insbesondere den Vorteil, dass sich die Struktur der Gewebeabschnitte mittels einer in dem chirurgischen Gewebefusionsinstrument enthaltenen optischen Sensoranordnung detektieren lässt.

In einer weiteren Ausgestaltung handelt es sich bei dem Zusatzstoff um einen gewebsdichtemarkierenden Zusatzstoff, welcher dazu ausgebildet ist, die Dichte der miteinander zu verbindenden biologischen Gewebeabschnitte oder von bereits miteinander verbundenen biologischen Gewebeabschnitten zu markieren, beispielsweise mittels Ultraschall. Ein geeigneter Zusatzstoff sind beispielsweise gasgefüllte Mikrobläschen ("microbubbles").

In einer weiteren Ausgestaltung handelt es sich bei dem Zusatzstoff um ein Röntgenkontrastmittel, wie beispielsweise Bariumsulfat oder ein iodhaltiges Kontrastmittel. Hierdurch ist mit besondrem Vorteil eine bildgebende Auswertung der miteinander zu verbindenden biologischen Gewebeabschnitte oder von bereits miteinander verbundenen biologischen Gewebeabschnitten möglich.

In einer weiteren Augestallung ist die körperliche Funktionsstruktur durch eine Formänderungsstruktur gebildet, die in Abhängigkeit von einer Änderung wenigstens eines physikalischen oder chemischen Umgebungsparameters aktivierbar ist. Die Formänderungsstruktur kann durch eine Formgedächtnisstruktur, durch eine schwammförmige Struktur, in Form eines Schrumpfschlauchs oder in anderer Art und Weise ausgeführt sein. Solange die Formänderungsstruktur nicht aktiviert ist, hat die Stützstruktur eine gleichbleibende, stabile Form.

In einer weiteren Ausgestaltung ist als Anderung von Umgebungsparametern eine auf die Funktionsstruktur wirkende Temperatur- und/oder Wassergehaltsänderung vorgesehen. Eine entsprechende Aktivierung kann demzufolge durch Wärmeeintrag oder auch durch Flüssigkeitszufuhr zu der Stützstruktur erfolgen.

In weiterer Ausgestaltung der Erfindung ist die körperliche Funktionsstruktur zumindest abschnittsweise elektrisch leitfähig ausgeführt. Bei elektrischer Leitfähigkeit der Funktionsstruktur kann die Funktionsstruktur zudem als elektrischer Pol zur elektrischen Aktivierung des Energieeintrags der Wärmeerzeugungsmittel ausgeführt sein. Bei dieser Ausgestaltung ist es ausreichend, wenn eine der beiden Greifstrukturen den anderen elektrischen Pol bildet, um bei Aktivierung einen elektrischen Energiefluss zu ermöglichen. Die Zufuhr elektrischer Energie kann außerdem eine gewünschte Formänderung der Funktionsstruktur bewirken, falls die Funktionsstruktur als Formänderungsstruktur gestaltet ist.

In einer weiteren Ausgestaltung ist die Stützstruktur als ein- oder beidseitig offener Hohlkörper ausgeführt, der wenigstens einen als Hohlorgan ausgeführten Gewebeabschnitt zumindest abschnittsweise außenseitig ummantelt. In weiterer Ausgestaltung weist die Formänderungsstruktur eine Schrumpfschlauchfunktion auf. Bei dieser Ausgestaltung kann die Stützstruktur eine außenseitige Stützung der Hohlorgane sowie eine sichere und dichte Verbindung zwischen diesen Hohlorganen bewirken.

In einer weiteren Ausgestaltung weist das chirurgische Gewebefusionsinstrument einen Sensor oder eine Sensoranordnung auf. Bei dem Sensor bzw. der Sensoranordnung kann es sich um einen elektronischen Sensor bzw. eine elektronische Sensoranordnung, Temperatursensor bzw. Temperatursensoranordnung oder um einen optischen Sensor, insbesondere spektroskopischen Sensor, bzw. eine optische Sensoranordnung, insbesondere spektroskopische Sensoranordnung, handeln. Vorzugsweise ist der Sensor bzw. die Sensoranordnung in einem Handgriff des chirurgischen Gewebefusionsinstruments integriert.

In einer weiteren Ausgestaltung weist das chirurgische Gewebefusionsinstrument eine Energiequelle, insbesondere einen Stromgenerator, vorzugsweise Hochfrequenzstromgenerator, einen Radiofrequenzgenerator, einen Ultraschallwellengenerator, einen Mikrowellengenerator oder eine Lichtquelle, insbesondere einen Laser, auf. Vorzugsweise ist die Energiequelle in einem Handgriff des chirurgischen Gewebefusionsinstruments integriert.

In weiterer Ausgestaltung der Erfindung weist das chirurgische Gewebefusionsinstrument ein Energieübertragungselement auf, welches dazu ausgebildet ist, einen Energieübertrag von einer Energiequelle des chirurgischen Gewebefusionsinstruments auf wenigstens eine der beiden Greifstrukturen, insbesondere auf beide Greifstrukturen, bevorzugt auf das Wärmeerzeugungsmittel/die Wärmeerzeugungsmittel, zu bewirken. Bei dem Energieübertragungselement kann es sich beispielsweise um elektrische Leitungen oder um Lichtleiter, d.h. transparente Bauteile, wie Fasern, Röhren oder Stäbe, welche Licht über kurze oder lange Strecken transportieren können, handeln.

In einer weiteren Ausgestaltung weist das chirurgische Gewebefusionsinstrument einen Akkumulator (Akku) auf. Der Akkumulator ist dazu ausgebildet, das Gewebefusionsinstrument, insbesondere eine darin integrierte Energiequelle, mit Strom zu versorgen. Bei dem Akkumulator kann es sich prinzipiell um eine Akkumulatorzelle (Akkuzelle) oder um einen Akkupack handeln. Der Akkumulator ist vorzugsweise (ebenfalls) in einem Handgriff des chirurgischen Gewebefusionsinstruments integriert.

Für die Stützstruktur wird die der Erfindung zugrundeliegende Aufgabe dadurch gelöst, dass wenigstens eine körperliche Funktionsstruktur, insbesondere wenigstens eine zusätzliche körperliche Funktionsstruktur, zur Unterstützung und Förderung einer Verbindung der biologischen Gewebeabschnitte vorgesehen ist, wie sie zuvor beschrieben wurden.

Weitere Vorteile und Merkmale der Erfindung ergeben sich aus den Ansprüchen.
- Fig. 1: zeigt in perspektivischer Darstellung eine Ausführungsform eines erfindungsgemäßen chirurgischen Gewebefusionsinstruments,
- Fig. 2: in vergrößerter Darstellung einen Kopfbereich des Gewebefusionsinstruments nach Fig. 1 in geöffneter Stellung,
- Fig. 3: in vergrößerter perspektivischer Darstellung eine Ausführungsform einer Stützstruktur für die Anordnung in dem Kopfbereich des Gewebefusionsinstruments gemäß Fig. 2,
- Fig. 4: eine Ausführungsform einer erfindungsgemäßen Stützstruktur in perspektivischer Darstellung,
- Fig. 5: eine Draufsicht auf die Stützstruktur nach Fig. 4,
- Fig. 6: eine Schnittdarstellung der Stützstruktur nach den Fig. 4 und 5,
- Fig. 7: in schematischer Schnittdarstellung die Einbindung der Stützstruktur nach den Fig. 4 bis 6 zwischen zwei biologische Gewebeabschnitte nach einer Gewebefusion mittels des Gewebefusionsinstruments nach den Fig. 1 und 2,
- Fig. 8: eine weitere Ausführungsform einer Stützstruktur in perspektivischer Darstellung,
- Fig. 9: eine Draufsicht auf die Stützstruktur nach Fig. 8,
- Fig. 10: in einer Schnittdarstellung gemäß der Schnittlinie X-X die Stützstruktur nach den Fig. 8 und 9,
- Fig. 11: in schematischer Schnittdarstellung die Einbindung der Stützstruktur nach den Fig. 8 bis 10 zwischen zwei miteinander zu verbindende Gewebeabschnitte mittels eines Gewebefusionsinstruments nach den Fig. 1 und 2,
- Fig. 12: in schematischer Darstellung den Kopfbereich des Gewebefusionsinstruments nach Fig. 2 mit einer weiteren Ausführungsform einer Stützstruktur, die zwischen die miteinander zu verbindenden Gewebeabschnitte eingefügt ist,
- Fig. 13: in schematischer Darstellung den Kopfbereich des Gewebefusionsinstruments nach Fig. 2 mit einer weiteren Ausführungsform einer Stützstruktur, die polygonförmig gestaltet ist und zwischen die Gewebeabschnitte einfügbar ist,
- Fig. 14: eine weitere schematische Darstellung des Kopfbereichs des Gewebefusionsinstruments nach Fig. 2 ähnlich den Fig. 12 und 13, wobei zwischen einen Ambossteil und einen Basisteil des Kopfbereichs eine weitere Ausführungsform einer Stützstruktur eingefügt ist, die eine Formänderungsstruktur aufweist,
- Fig. 15: die Ausführung gemäß Fig. 14 mit schematischer Darstellung der erfolgten Formänderung der Stützstruktur,
- Fig. 16: schematisch eine weitere Ausführungsform eines Gewebefusionsinstruments mit einer die Gewebeabschnitte außen ummantelnden Stützstruktur, die gemäß
- Fig. 17: eine Formänderung nach Art eines Schrumpfschlauchs erfährt.

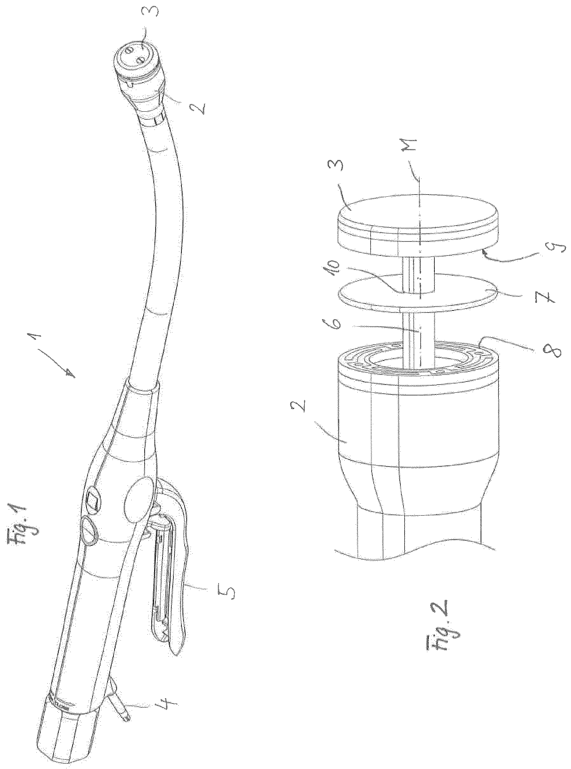

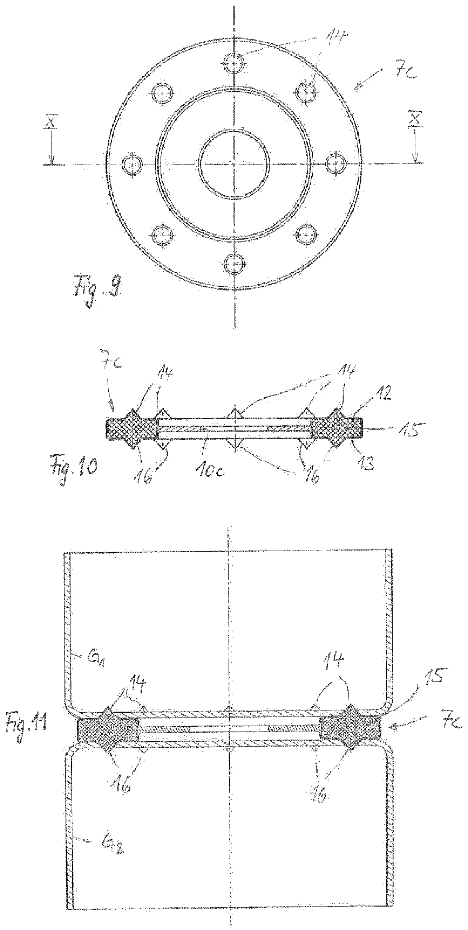

Ein chirurgisches Gewebefusionsinstrument nach den Fig. 1 bis 17 ist als zirkuläres Gewebefusionsinstrument 1 ausgeführt. Das Gewebefusionsinstrument 1 weist einen nicht näher bezeichneten Griffbereich auf, der für einen Betrieb des Gewebefusionsinstruments von Hand ergreifbar ist. Dem Griffbereich ist eine Betätigungshandhabe 5 zugeordnet, die eine Aktivierung und Steuerung des Gewebefusionsinstruments 1 ermöglicht. Das Gewebefusionsinstrument 1 wird durch ein mit dem Bezugszeichen 4 angedeutetes Stromkabel mit Strom versorgt. Alternativ kann das Gewebefusionsinstrument 1 mittels eines Akkumulators mit Strom versorgt werden. Die Verwendung eines Akkumulators erlaubt mit besonderem Vorteil den Einbau einer Energiequelle, insbesondere eines Hochfrequenzstromgenerators, in das chirurgische Gewebefusionsinstrument 1. Bevorzugt sind in diesem Fall die Energiequelle sowie der Akkumulator in dem Griffbereich des chirurgischen Gewebefusionsinstruments 1 integriert. Von dem Griffbereich aus ragt über einen längserstreckten Hals ein Kopfbereich des Gewebefusionsinstruments 1 ab, der einen Basisteil 2 sowie einen Ambossteil 3 aufweist. Der Ambossteil 3 ist mittels eines Ambossschafts 6 koaxial zu einer Mittellängsachse M des Halses des Kopfbereichs in dem Basisteil 2 längsverschiebbar gelagert. Mittels eines nicht näher dargestellten Antriebssystems ist der Ambossteil 3 entlang der Mittellängsachse M zwischen einer Öffnungsstellung gemäß Fig. 2 und einer Schließstellung gemäß Fig. 1 relativ zu dem Basisteil 2 verfahrbar. Der Basisteil 2 und der Ambossteil 3 weisen einander zugewandte Stirnflächen auf, die Kontaktflächen zum Ergreifen und Zusammenführen von miteinander zu verbindenden biologischen Gewebeabschnitten bilden. Sowohl der Ambossteil 3 als auch der Basisteil 2 definieren eine Greifstruktur im Sinne der Erfindung.

Die beiden Stirnflächen des Basisteils 2 und des Ambossteils 3 sind mit Elektrodenanordnungen 8, 9 versehen, die als Wärmeerzeugungsmittel dazu dienen, elektrisch beaufschlagt zu werden und so über hochfrequente elektromagnetische Wellen einen Wärmeeintrag auf die miteinander zu verbindenden biologischen Gewebeabschnitte einzubringen. Der zwischen den Kontaktflächen des Basisteils 2 und des Ambossteils 3 in der Schließstellung definierte Kontaktbereich wird als Verbindungsbereich oder als Verbindungsstelle für die Gewebeabschnitte bezeichnet. Die Elektrodenanordnungen 8, 9 ermöglichen einen Energieeintrag fokussiert und gerichtet auf den Verbindungsbereich, so dass ein hoher Wärmeeintrag in die miteinander zu verbindenden Gewebeabschnitte einbringbar ist, der eine gewünschte Gewebefusion zwischen den Gewebeabschnitten bewirkt. Hierdurch ergibt sich eine stoffschlüssige Verbindung zwischen den Gewebeabschnitten, die mechanisch durch zusätzliche mechanische Verbindungsmittel wie Klammern oder Ähnliches unterstützt oder verstärkt werden kann. Mittels des zirkulären Gewebefusionsinstruments 1 werden insbesondere biologische Gewebeabschnitte miteinander verbunden, die als Hohlorgane ausgeführt sind.

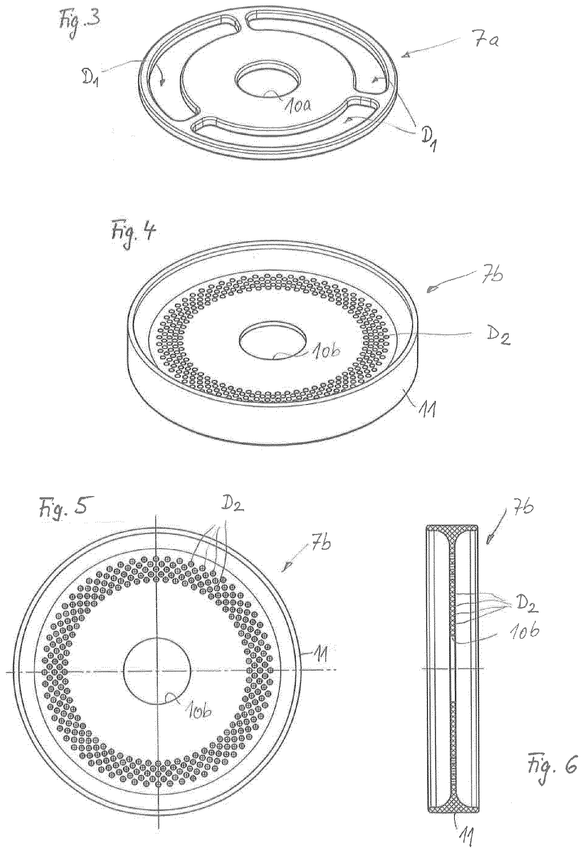

Zur Unterstützung und Förderung der Verbindung zwischen den Gewebeabschnitten ist dem Kopfbereich des Gewebefusionsinstruments 1 zusätzlich eine Stützstruktur 7, 7a bis 7g zugeordnet, die zumindest weitgehend rotationssymmetrisch gestaltet und die Mittellängsachse M des Kopfbereichs koaxial umgibt. Die Stützstruktur 7 gemäß Fig. 2 stellt lediglich eine schematische Darstellung dar nach Art eines Platzhalters für die anhand der Fig. 3 bis 17 detailliert dargestellten Stützstrukturen 7a bis 7g. Die schematisch dargestellte Stützstruktur 7 zeigt die Anordnung der Stützstrukturen 7a bis 7f im Kopfbereich des Gewebefusionsinstruments 1.

Alle Ausführungsformen von Stützstrukturen 7, 7a bis 7g sind aus einem medizinisch verträglichen Material gestaltet oder weisen ein solches Material auf und sind in einem unbelasteten Ausgangszustand bei geöffnetem Gewebefusionsinstrument 1 gemäß Fig. 2 formstabil ausgeführt. Jede der Stützstrukturen 7, 7a bis 7g dient zur Förderung und Unterstützung der Gewebefusion zwischen den Gewebeabschnitten G₁, G₂. Alle Stützstrukturen 7a bis 7g weisen eine körperliche Funktionsstruktur, insbesondere eine zusätzliche körperliche Funktionsstruktur, auf. Bei den Ausführungsformen gemäß den Fig. 3 bis 11 wird die körperliche Funktionsstruktur durch mechanische Funktionsabschnitte gebildet. Alle Stützstrukturen 7, 7a bis 7g sind vorzugsweise aus einem resorbierbaren Material gestaltet oder weisen ein solches Material auf.

Die Stützstruktur 7a nach Fig. 3 weist kreisbogenförmig längs erstreckte Durchtritte D₁ auf, die gleichmäßig über den Umfang der Stützstruktur 7a verteilt sind. Die Stützstruktur 7a ist scheibenförmig gestaltet und weist ein zentrales Aufnahmeauge 10a auf, das zur Lagerung der Stützstruktur 7a auf dem Ambossschaft 6 des Ambossteils 3 dient. Die Durchtritte D₁ sind fluchtend zu den Elektrodenanordnungen 8 und 9 des Ambossteils 3 und des Basisteils 2 positioniert und befinden sich demzufolge an den Stellen des höchsten Energieeintrags. Durch die Durchtritte D₁ hindurch werden die Gewebeabschnitte G₁, G₂ bei einer Gewebefusion miteinander verbunden, wobei sie die Stützstruktur 7a sandwichartig zwischen sich einschließen.

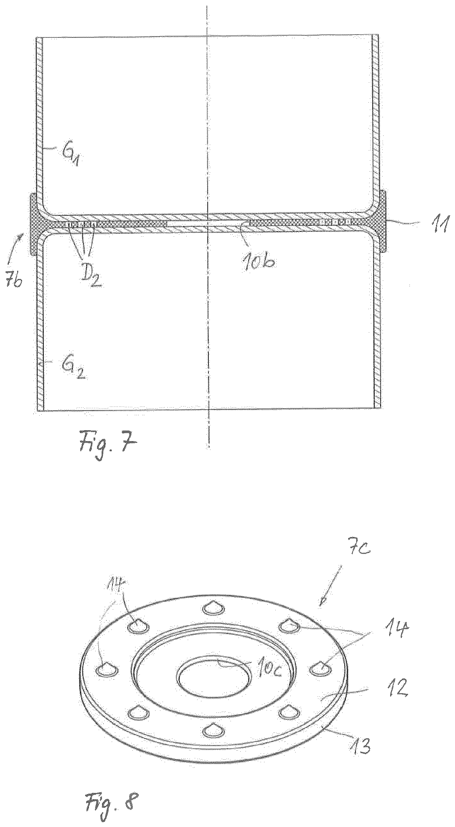

Bei der erfindungsgemäßen Ausführungsform nach Fig. 4 weist die Stützstruktur 7b ebenfalls Durchtritte D₂ auf, die erfindungsgemäß als Perforationen gestaltet sind. Auch die als Perforationen gestalteten Durchtritte D₂ erstrecken sich über einen Umfang der Stützstruktur 7b, die als Ringscheibe gestaltet ist. Die Stützstruktur 7b weist ein Aufnahmeauge 10b analog der zuvor beschriebenen Stützstruktur 7a auf. Die Durchtritte D₂ sind gleichmäßig über den Umfang der Stützstruktur 7b kreisförmig verteilt und sind fluchtend zu den Elektrodenanordnungen 8, 9 angeordnet, um im Bereich des höchsten Energieeintrags eine besonders gute Verbindung zwischen den Gewebeabschnitten G₁, G₂ erzielen zu können. Die Stützstruktur 7b ist zusätzlich mit einem Randsteg 11 versehen, der einstückig am Außenumfang der ringscheibenförmigen Stützstruktur 7b angeformt ist und axial zu einer Radialebene der Stützstruktur 7b zu gegenüberliegenden Seiten relativ zu einer Scheibenfläche der Stützstruktur 7b abragt. Der Randsteg 11 flankiert die Gewebeabschnitte G₁, G₂ nach erfolgter Gewebefusion radial außenseitig im Bereich der Verbindungsstelle zwischen den Gewebeabschnitten G₁, G₂. Der Randsteg 11 trägt demzufolge zur Versteifung und Stützung der Verbindungsstelle zwischen den Gewebeabschnitten G1, G2 bei. Anhand der Fig. 7 ist erkennbar, dass die Stützstruktur 7b sandwichartig zwischen die Gewebeabschnitte G₁, G₂ eingefügt ist, die als Hohlorgane ausgeführt sind. Die Verbindungsstelle erstreckt sich radial zu einer imaginären Mittellängsachse der miteinander fluchtenden, als Hohlorgane ausgebildeten Gewebeabschnitte G₁, G₂ als flächige Verbindung im Bereich der durch die Stützstruktur 7b vorgegebenen Radialebene.

Die Stützstruktur 7c nach den Fig. 8 bis 11 ist ebenfalls scheibenringförmig ausgeführt mit einem zentralen Aufnahmeauge 10c zur Lagerung auf dem Ambossschaft 6 des Ambossteils 3. Die Stützstruktur 7c weist an ihren gegenüberliegenden Stirnflächen als zusätzliche körperliche Funktionsstrukturen Profilierungen 14, 16 auf, die als höckerartige Spitzen von der Stirnfläche axial nach außen abragen. Die Profilierungen 14, 16 ragen von den gegenüberliegenden Stirnflächen der Stützstruktur 7c nach außen ab.

Die Stützstruktur 7c ist aus zwei Ringschalen 12 und 13 aufgebaut, die an ihren Randbereichen dicht miteinander verbunden sind. Zwischen den Ringschalen 12 und 13 ist ein Speicherraum 15 zur Aufnahme eines flüssigen oder fließfähigen Zusatzstoffs vorgesehen, der dazu dient, im Bereich der Verbindungsstelle zwischen den Gewebeabschnitten G₁, G₂ die Gewebefusion zu unterstützen oder zu fördern. Der flüssige oder fließfähige Zusatzstoff ist medizinisch verträglich und kann insbesondere Klebstoffeigenschaften oder Wundheilungsfördereigenschaften aufweisen.

Die den Speicherraum 15 umgebenden Stirn- und Seitenwandungen der Ringschalen 12, 13 sind derart ausgeführt, dass sie bei Druckbelastung auf die Stirnflächen platzen oder aufreißen können. Hierdurch kommt der Zusatzstoff frei und kann sich im Bereich der Verbindungsstelle zwischen den Gewebeabschnitten G₁, G₂ verteilen. Die Profilierungen 14, 16 deformieren sich bei einer Druckbeaufschlagung während eines Schließvorgangs des Kopfbereichs des Gewebefusionsinstruments 1 und unterstützen das Aufreißen oder Aufplatzen der Wandungen des Speicherraums 15.

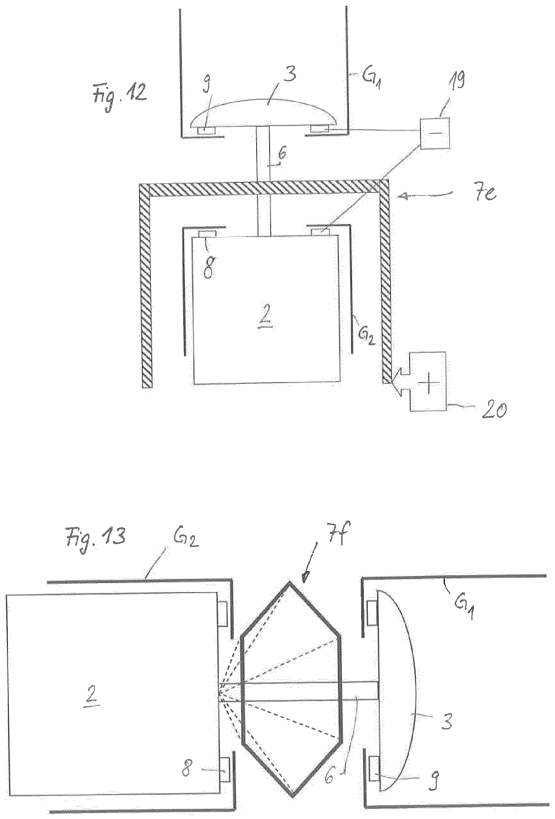

Bei der Ausführungsform nach Fig. 12 ist die Stützstruktur 7e im Kopfbereich des Gewebefusionsinstruments becherförmig gestaltet, wobei sie einen radial zur Mittellängsachse des Kopfbereiches erstreckten, scheibenförmigen Bereich sowie eine axial erstreckte Ringwandung aufweist, die den Basisteil 2 und den zugeordneten Gewebeabschnitt G₂ außenseitig umgibt. Das Material der becherförmigen Stützstruktur 7e ist zumindest abschnittsweise elektrisch leitend ausgeführt. Es wird für einen entsprechenden Wärmeeintrag auf die Gewebeabschnitte G₁, G₂ mittels elektrischer Energiebeaufschlagung als Pluspol 20 eingesetzt, wohingegen die Elektrodenanordnungen 8 und 9 als Minuspol 19 gesteuert sind.

Bei der Ausführungsform nach Fig. 13 ist die Stützstruktur 7f aus einem scheibenförmigen Ausgangszustand analog Fig. 2 bereits in einen dreidimensionalen, polygonen Formänderungszustand aufgerichtet. Hierzu weist die Stützstruktur 7f vorzugsweise ein Formgedächtnismaterial auf, das insbesondere durch Temperaturbeaufschlagung, Beaufschlagung mit elektrischer Energie oder durch Feuchtigkeitsbeaufschlagung aktiviert wird. Die Form, in die die Stützstruktur 7f sich aufrichten kann, ist abhängig vom jeweiligen Einsatzzweck. In aufgerichteter Form kann die Stützstruktur sich entweder innen an die Gewebeabschnitte G₁, G₂ anlegen oder diese außenseitig im Bereich der Verbindungsstelle ummanteln.

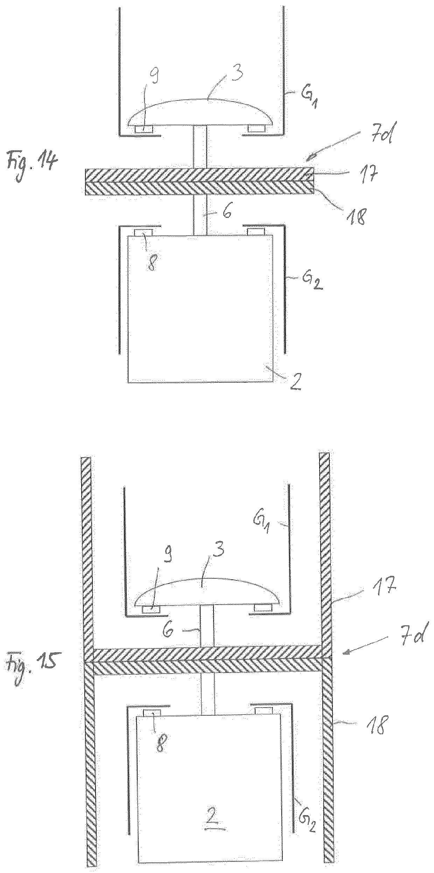

Bei der Ausführungsform nach den Fig. 14 und 15 ist die Stützstruktur 7d in einem Ausgangszustand als zweiteilige Ringscheibe mit einem ersten Ringteil 17 und einem zweiten Ringteil 18 versehen, die in der Radialebene flächig aneinandergefügt sind. Auch die Stützstruktur 7d ist mit einer Formänderungseigenschaft versehen, wobei die beiden Teile 17 und 18 sich nach Aktivierung in gegenläufig zueinander ausgerichtete becher- oder hülsenartige Formen ausrichten, wie Fig. 15 zu entnehmen ist. In ihrem formgeänderten Zustand umgeben die Teile 17 und 18 die Gewebeabschnitte G₁, G₂ mit ihren Ringwandungen über eine bestimmte axiale Länge radial außenseitig, so dass die Stützstruktur 7d neben einer radialen Flächenverbindung mit den Gewebeabschnitten G₁, G₂ im Bereich der radialen Verbindungsstelle auch eine außenseitige Ummantelung über eine bestimmte axiale Länge definiert.

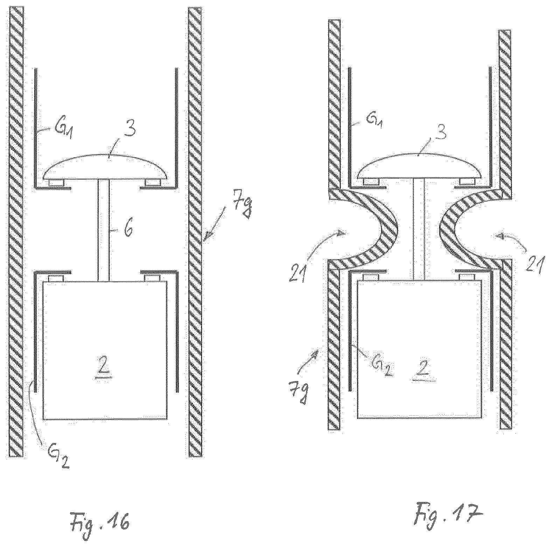

Bei der Ausführungsform nach den Fig. 16 und 17 ist die Stützstruktur 7g schlauchförmig gestaltet und ummantelt die Gewebeabschnitte G₁, G₂ und den Kopfbereich des Gewebefusionsinstruments radial außenseitig. Auch die Stützstruktur 7g ist mit einer Formänderungsstruktur versehen. Aktiviert durch einen physikalischen oder chemischen Umgebungsparameter, insbesondere durch Temperaturbeaufschlagung, wird die Formänderungsstruktur aus einem Ausgangszustand gemäß Fig. 16 im Verbindungsbereich zwischen dem Ambossteil 3 und dem Basisteil 2 geschrumpft, wodurch sich ein ringförmiger Schrumpfeinzug 21 ergibt, der bei einem Zusammenführen von Ambossteil 3 und Basisteil 2 zwischen die Gewebeabschnitte G₁, G₂ axial eingepresst wird.

## Patentansprüche

1. Zirkuläres chirurgisches Gewebefusionsinstrument (1) mit zwei relativ zueinander beweglichen Greifstrukturen (2, 3), die für das Zusammenführen von miteinander zu verbindenden biologischen Gewebeabschnitten (G₁, G₂) gestaltet sind, mit Wärmeerzeugungsmitteln (8, 9), die den Greifstrukturen (2, 3) zugeordnet sind und bei einer Gewebefusion einen Wärmeeintrag im Bereich einer Verbindungsstelle der biologischen Gewebeabschnitte (G₁, G₂) bewirken, sowie mit einer Stützstruktur (7, 7a bis 7g), die den Greifstrukturen zugeordnet ist und bei einer Gewebefusion mit den Gewebeabschnitten (G₁, G₂) in Wirkverbindung gelangt, wobei die Stützstruktur (7, 7a bis 7g) wenigstens eine zusätzliche körperliche Funktionsstruktur zur Unterstützung oder Förderung der Gewebefusion aufweist, wobei die Greifstrukturen (2, 3) in Form eines Basisteils (2) sowie eines Ambossteils (3) ausgebildet sind, wobei ein von einem Griffbereich des Gewebefusionsinstruments (1) über einen längserstreckten Hals ragender Kopfbereich den Basisteil (2) sowie den Ambossteil (3) aufweist, der Ambossteil (3) mittels eines Ambossschafts (6) koaxial zu einer Mittellängsachse (M) des Halses des Kopfbereichs in dem Basisteil (2) längsverschiebbar gelagert ist, wobei der Ambossteil (3) entlang der Mittellängsachse M zwischen einer Öffnungsstellung und einer Schließstellung relativ zu dem Basisteil (2) verfahrbar ist, und der Basisteil (2) und der Ambossteil (3) einander zugewandte Stirnflächen aufweisen, die Kontaktflächen zum Ergreifen und Zusammenführen der miteinander zu verbindenden Gewebeabschnitte (G1, G2) aufweisen, wobei die beiden Stirnflächen des Basisteils (2) und des Ambossteils (3) mit Elektrodenanordnungen (8, 9) versehen sind, die als Wärmeerzeugungsmittel dazu dienen, elektrisch beaufschlagt zu werden, wobei die Stützstruktur (7b) Durchtritte (D₂), die als Perforationen gestaltet sind, aufweist, wobei sich die als Perforationen gestalteten Durchtritte (D₂) über einen Umfang der Stützstruktur (7b) erstrecken, gleichmäßig über den Umfang der Stützstruktur (7b) kreisförmig verteilt und fluchtend zu den Elektrodenanordnungen (8, 9) angeordnet sind, wobei die Stützstruktur (7b) als Ringscheibe gestaltet ist, ein Aufnahmeauge (10b), das zur Lagerung der Stützstruktur (7b) auf dem Ambossschaft (6) des Ambossteils (3) dient, aufweist **dadurch gekennzeichnet, dass** die Stützstruktur (7b) zusätzlich mit einem Randsteg (11) versehen ist, der einstückig am Außenumfang der ringscheibenförmigen Stützstruktur (7b) angeformt ist und axial zu einer Radialebene der Stützstruktur (7b) zu gegenüberliegenden Seiten relativ zu einer Scheibenfläche der Stützstruktur (7b) abragt.

2. Zirkuläres chirurgisches Gewebefusionsinstrument (1) nach Anspruch 1, wobei die körperliche Funktionsstruktur zumindest abschnittsweise elektrisch leitfähig ausgeführt ist.

3. Zirkuläres chirurgisches Gewebefusionsinstrument (1) nach Anspruch 2, wobei die körperliche Funktionsstruktur als elektrischer Pol (20) zur elektrischen Aktivierung des Energieeintrags der Wärmeerzeugungsmittel ausgeführt ist.

## Claims

1. Circular surgical tissue fusion instrument (1) with two gripping structures (2, 3) which are movable relative to each other and which are designed to bring together biological tissue sections (G₁, G₂) that are to be connected to each other, with heat-generating means (8, 9) which are assigned to the gripping structures (2, 3) and, during tissue fusion, cause heat to be introduced in the area of a connection site of the biological tissue sections (G₁, G₂), and with a support structure (7, 7a to 7g) which is assigned to the gripping structures and, during tissue fusion, is operatively connected to the tissue sections (G₁, G₂), wherein the support structure (7, 7a to 7g) has at least one additional physical functional structure for aiding or promoting the tissue fusion, wherein the gripping structures (2, 3) are in the form of a base part (2) and an anvil part (3), wherein a head area protruding from a grip area of the tissue fusion instrument (1) via an elongate neck has the base part (2) and the anvil part (3), the anvil part (3) is mounted longitudinally displaceably in the base part (2) by means of an anvil shaft (6) coaxial to a central longitudinal axis (M) of the neck of the head area, wherein the anvil part (3) can travel relative to the base part (2) along the central longitudinal axis (M) between an open position and a closed position, and the base part (2) and the anvil part (3) have end faces which are directed toward each other, which form contact faces by which tissue sections (G₁, G₂) to be connected to each other are grasped and brought together, wherein the two end faces of the base part (2) and the anvil part (3) are provided with electrode arrangements (8, 9) which, as heat-generating means, are acted upon electrically, wherein the support structure (7b) has apertures (D₂) which are designed as perforations, wherein the apertures (D₂) designed as perforations extend about a circumference of the support structure (7b), are distributed uniformly in the shape of a circle about the circumference of the support structure (7b) and are arranged flush with the electrode arrangements (8, 9), wherein the support structure (7b) is designed as an annular disk, has a receiving eye (10b) for mounting the support structure (7b) on the anvil shaft (6) of the anvil part (3),
**characterized in that**
the support structure (7b) is additionally provided with an edge web (11), is formed integrally on the outer periphery of the annular disk-shaped support structure (7b) and protrudes axially, with respect to a radial plane of the support structure (7b), on opposite sides relative to a disk surface of the support structure (7b).

2. Circular surgical tissue fusion instrument (1) according to claim 1, wherein the physical functional structure is designed to be electrically conductive at least in parts.

3. Circular surgical tissue fusion instrument (1) according to claim 2, wherein the physical functional structure is designed as an electrical pole (20) for electrical activation of the energy input by the heat-generating means.

## Revendications

1. Instrument chirurgical circulaire de fusion tissulaire (1), comprenant deux structures de préhension (2, 3) déplaçables l'une par rapport à l'autre, qui sont configurées pour l'assemblage de portions tissulaires biologiques (G₁, G₂) devant être raccordées ensemble, des moyens de génération de chaleur (8, 9) qui sont associés aux structures de préhension (2, 3), et qui, lors d'une fusion tissulaire, produisent un apport de chaleur dans la région d'une zone de raccordement des portions tissulaires biologiques (G₁, G₂), et une structure de support (7, 7a à 7g), qui est associée aux structures de préhension, et qui vient en liaison fonctionnelle avec les portions tissulaires (G₁, G₂) dans le cas d'une fusion tissulaire, la structure de support (7, 7a à 7g) présentant au moins une structure fonctionnelle physique supplémentaire pour supporter ou favoriser la fusion tissulaire, les structures de préhension (2, 3) étant réalisées sous la forme d'une partie de base (2) et d'une partie d'enclume (3), une région de tête faisant saillie depuis une région de préhension de l'instrument de fusion tissulaire (1) au-delà d'un col allongé présentant la partie de base (2) et la partie d'enclume (3), la partie d'enclume (3) étant supportée de manière déplaçable longitudinalement au moyen d'une tige d'enclume (6) coaxialement à un axe médian longitudinal (M) du col de la région de tête dans la partie de base (2), la partie d'enclume (3) pouvant être déplacée le long de l'axe médian longitudinal (M) entre une position d'ouverture et une position de fermeture par rapport à la partie de base (2), et la partie de base (2) et la partie d'enclume (3) présentant des surfaces frontales tournées l'une vers l'autre qui présentent des surfaces de contact pour saisir et assembler les portions tissulaires (G₁, G₂) devant être raccordées ensemble, les deux surfaces frontales de la partie de base (2) et de la partie d'enclume (3) étant pourvues d'agencements d'électrodes (8, 9) qui, en tant que moyens de génération de chaleur, servent à être sollicités électriquement, la structure de support (7b) présentant des passages (D₂) qui sont configurés sous forme de perforations, les passages (D₂) configurés sous forme de perforations s'étendant sur une périphérie de la structure de support (7b), étant répartis uniformément en cercle sur la périphérie de la structure de support (7b) et étant disposés en affleurement avec les agencements d'électrodes (8, 9), la structure de support (7b) étant configurée sous forme de disque annulaire, présentant un œillet de réception (10b), qui sert à supporter la structure de support (7b) sur la tige d'enclume (6) de la partie d'enclume (3),
**caractérisé en ce que** la structure de support (7b) est en outre pourvue d'une nervure marginale (11) qui est façonnée d'une seule pièce à la périphérie extérieure de la structure de support (7b) en forme de disque annulaire et fait saillie axialement par rapport à un plan radial de la structure de support (7b) vers des côtés opposés par rapport à une surface de disque de la structure de support (7b).

2. Instrument chirurgical circulaire de fusion tissulaire (1) selon la revendication 1, dans lequel la structure fonctionnelle physique est réalisée au moins en partie sous forme électriquement conductrice.

3. Instrument chirurgical circulaire de fusion tissulaire (1) selon la revendication 2, dans lequel la structure fonctionnelle physique est réalisée sous forme de pôle électrique (20) pour l'activation électrique de l'apport d'énergie des moyens de génération de chaleur.
